# EUROPEAN PATENT APPLICATION

(11) **EP 4 563 708 A1**
(43) Date of publication of application: **04.06.2025**
(21) Application number: 22953039.9
(22) Date of filing: 26.07.2022
(51) Int. Cl.: C12Q 1/6844, C12M 1/00, G01N 35/00

(54) **GENETIC TESTING METHOD AND GENETIC TESTING DEVICE**

(71) Applicant: HITACHI HIGH-TECH CORPORATION, Tokyo 105-6409 (JP)
(72) Inventor: AKUTSU Masashi, Tokyo 105-6409 (JP)
(74) Representative: MERH-IP Matias Erny Reichl Hoffmann Patentanwälte PartG mbB
(86) International application number: PCT/JP2022/028838
(87) International publication number: WO 2024/023944

(57) **Abstract**

A genetic testing device 1 further comprises a calculation unit 2d that adds, to the time at which an analyte is put, an available standby time depending on the priority of the analyte and calculates a target time by which dispensation to the analyte is to be completed. A control unit 2c executes dispensation of analytes in the order of target time. Consequently, in the genetic testing method and genetic testing device provided by the present invention, priority is given to measurement of an analyte with high priority, and measurement of an analyte with low priority is prevented from being overly delayed when analytes with high priority are introduced in succession.

## Description

### Technical Field

The present invention relates to a genetic testing method and a genetic testing device for extracting nucleic acid from a biological sample and amplifying genes using a polymerase chain reaction (hereinafter abbreviated as PCR).

### Background Art

As an example of a sample processing device that prevents deterioration of samples and reagents due to an increase in a waiting time other than reactions and improves the operating efficiency of a device, PTL 1 discloses a device that includes a plurality of reaction parts, at least two of which independently react samples, an input/output terminal for inputting an upper limit of a waiting time other than for a reaction of the sample and a reaction time of the sample, a schedule management unit that determines one or more of the time of feeding of a sample, a reaction part to be used to process the sample, and the waiting time other than the reaction of the sample on the basis of the upper limit of the waiting time other than the reaction and the reaction time which are input in the input/output terminal, and an overall control unit that performs control so that the sample is processed in the plurality of reaction parts on the basis of the determination performed by the schedule management unit.

### Citation List

### Patent Literature

PTL 1: JP2008-039556A

### Summary of Invention

### Technical Problem

A PCR method is used in devices that analyze nucleic acid contained in samples (so-called biological samples) derived from organisms such as blood and urine. The PCR method is a technique that uses a heat-resistant polymerase and primers to amplify a target nucleic acid by increasing or decreasing the temperature, and is widely used in fields of genetic engineering, biological testing, biological detection, and the like.

The principle of a PCR is to amplify a target DNA exponentially by repeating cycles according to a thermal profile (an increase and a decrease in temperature) many times.

Quantitative testing methods to which such a PCR method is applied include a real-time PCR or a quantitative polymerase chain reaction (hereinafter abbreviated as qPCR). The qPCR method is a highly sensitive genetic analysis method, and is being increasingly applied to clinical testing such as quantitative gene expression analysis, pathogen detection, and drug discovery target verification.

Genetic testing using these techniques requires approximately two hours because processes from an extraction process to a PCR measurement process is performed in a batch. Furthermore, as genetic testing using these techniques, a batch processing method in which a plurality of biological samples are installed in a batch and processed is generally used.

In recent years, these genetic testing devices have become increasingly widespread, mainly in university hospitals and clinical testing centers that need to process biological samples from a large number of patients in a short period of time, but within these hospitals, speeding up diagnosis for patients who require emergency treatment is an issue. In addition, for items such as airborne infections that are transmitted through the air, when the results cannot be determined promptly, it could lead to secondary infections within the hospital, and thus there is an extremely strong demand for speeding up diagnosis required to take prompt action.

In response to these backgrounds, the need for rapid measurement of urgent samples in genetic testing devices is increasing.

Prior art has proposed attempts to promptly measure urgent samples. For example, PTL 1 proposes setting an upper limit on a waiting time other than for sample reactions and performing control so that the samples are carried within the time.

However, in actual operations in hospitals and the like, blood of samples is drawn or collected in advance, and the samples are fed into a device at any time after they arrive at a laboratory. That is, it can be said that it is difficult for the device to control the timing when the sample should be fed as a main body.

As described above, as a genetic testing device, a batch processing system that processes a plurality of biological samples in a batch is generally used. This is to ensure the number of measurements per unit time by performing the same measurement a plurality of times at the same time because a single measurement takes several hours. In the case of operations for the purpose of research such as drug discovery, the priority of each sample is the same and items to be measured are generally the same, and thus this method does not pose a major problem.

However, when biological samples are measured from unspecified patients, priority may be different for each sample, and test items may be also mainly different. Furthermore, the time when a sample arrives at a laboratory and becomes ready for measurement is different for each sample.

Thus, it is necessary to perform processing according to priority corresponding to a sample and a test item. In particular, it is necessary to perform measurement by prioritizing the measurement of urgent samples that are determined to have a high priority.

However, when a priority process is performed simply by focusing only on the priority of a sample, there is a concern that the processing of low-priority samples will be delayed continuously when high-priority samples are fed successively, and results will not be obtained for a long time.

Furthermore, in such genetic testing devices for hospitals, a portion where a sample is fed is configured such that only the feeding of the sample is performed. That is, an operation is performed such that a laboratory technician simply feeds arriving samples in order. On the other hand, the inside of the device has a structure an extraction process is performed collectively on a plurality of samples. Further, in a PCR process, a measurement process is performed collectively on a plurality of samples. That is, there is also a problem that the overall efficiency will decrease when the measurement process is not performed collectively on a plurality of items.

Furthermore, since PCR measurement takes several hours, leaving a sample at room temperature for an excessively long period of time can lead to deterioration such as evaporation of the sample, which is not preferable.

Thus, although a fed sample should be processed promptly, it is necessary to perform a measurement process while taking the priority of the sample and the operating efficiency of equipment into consideration.

The present invention provides a genetic testing method and a genetic testing device which are capable of prioritizing the measurement of high-priority samples and avoiding having to continuously wait for the measurement of low-priority samples even when the high-priority samples are fed successively.

### Solution to Problem

The present invention includes a plurality of means for solving the above problems. One example is a genetic testing method including a feeding step of feeding a sample, a pretreatment step of acquiring biomolecules including a measurement target from the sample fed in the feeding step, a measurement step of measuring the biomolecules acquired in the pretreatment step, an analysis data processing step of performing an analysis calculation process based on a measurement result in the measurement step, and a calculation step of calculating a target time by which aliquoting of the sample is to be completed by adding a waiting time according to priority of the sample to a time when the sample is fed, in which an aliquoting process for the sample is performed in the order of the target time.

### Advantageous Effects of Invention

According to the present invention, it is possible to prioritize the measurement of high-priority samples and avoiding having to continuously wait for the measurement of low-priority samples even when the high-priority samples are fed successively. Problems, configurations, and effects other than those described above will become apparent from the following description of examples.

### Brief Description of Drawings

[Fig. 1] Fig. 1 is a diagram showing one embodiment of a genetic testing device to which the present invention is applied.
[Fig. 2] Fig. 2 is a diagram showing a setting screen for an aliquoting waiting sample used in one example of the genetic testing device to which the present invention is applied.
[Fig. 3] Fig. 3 is a diagram showing a sample and a sample rack used in one example of the genetic testing device to which the present invention is applied.
[Fig. 4] Fig. 4 shows an example of a flowchart for calculating an aliquoting end target in one example of the genetic testing device to which the present invention is applied.
[Fig. 5]Fig. 5 is a diagram showing an example of an aliquoting order management table in one example of the genetic testing device to which the present invention is applied.
[Fig. 6] Fig. 6 is a diagram showing an example of an aliquoting order management table in one example of the genetic testing device to which the present invention is applied.
[Fig. 7] Fig. 7 is a diagram showing an example of an aliquoting order management table in one example of the genetic testing device to which the present invention is applied.

### Description of Embodiments

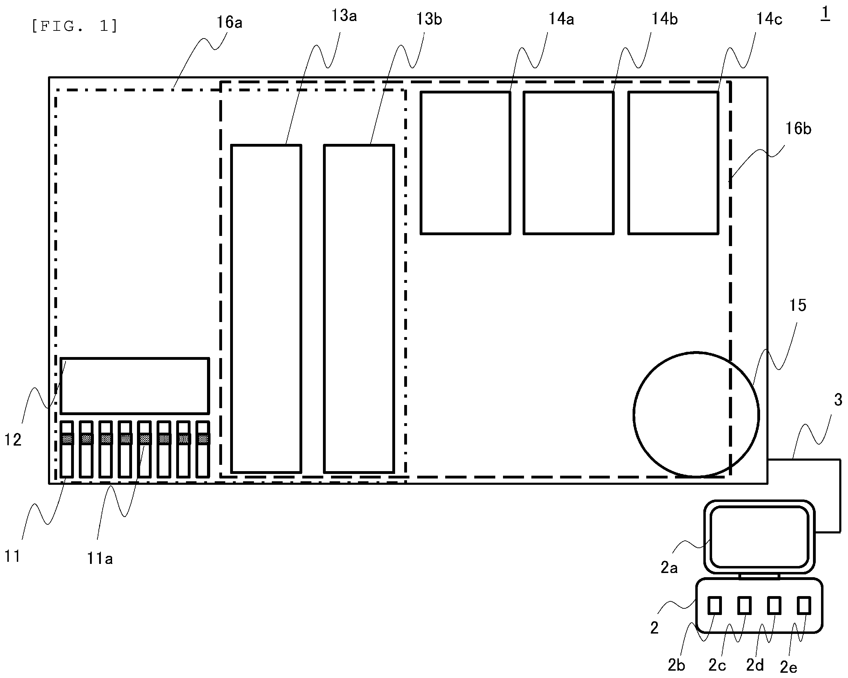

An example of a genetic testing method and a genetic testing device of the present invention will be described with reference to Figs. 1 to 7. In the drawings used in this specification, the same or corresponding components are denoted by the same or similar reference numerals, and repeated description of these components may be omitted.

First, the overall configuration of the genetic testing device will be described with reference to Fig. 1. Fig. 1 is a block diagram showing an overview of the genetic testing device of this example. Here, a genetic testing device 1 that transports a sample rack loaded with a plurality of sample containers is assumed.

The genetic testing device 1 shown in Fig. 1 includes a sample rack feeding part 11, a sample rack buffer part 12, a first aliquoting part 16a, nucleic acid extraction parts 13a and 13b, a second aliquoting part 16b, amplification parts 14a, 14b and 14c, a control computer 2, and the like.

The sample rack feeding part 11 is a part for feeding and collecting a sample rack, and includes a barcode reader 11a that reads the priority of a sample when the sample is fed. The sample rack feeding part 11 preferably executes a feeding step of feeding a sample, and the barcode reader 11a preferably executes a reading step of reading the priority of a sample when the sample is fed.

The sample rack buffer part 12 is a part that temporarily keeps one or more sample racks that hold sample containers containing samples awaiting analysis which are fed from the sample rack feeding part 11, and temporarily keeps one or more sample racks before being collected.

The first aliquoting part 16a is a part that aliquots samples from sample containers placed in the sample rack on the sample rack feeding part 11 or the sample rack buffer part 12 to the nucleic acid extraction parts 13a and 13b. The first aliquoting part 16a includes a drive mechanism in the X-axis, Y-axis, and Z-axis directions and an aliquoting probe, and moves above the sample rack feeding part 11, the sample rack buffer part 12, and the nucleic acid extraction parts 13a and 13b. The first aliquoting part 16a preferably executes a first aliquoting step of aliquoting a sample from a feeding step to a pretreatment step.

The nucleic acid extraction parts 13a and 13b are parts that acquire biomolecules including a measurement target from the sample fed into the sample rack feeding part 11. The nucleic acid extraction parts 13a and 13b preferably execute the pretreatment step of acquiring the biomolecules including the measurement target from the sample that is fed in the feeding step.

The second aliquoting part 16b is a part that aliquots biomolecules from the nucleic acid extraction parts 13a and 13b to the amplification parts 14a, 14b, and 14c. The second aliquoting part 16b includes a driving mechanism in the X-axis, Y-axis, and Z-axis directions and an aliquoting probe, similar to the first aliquoting part 16a, and moves above the nucleic acid extraction parts 13a and 13b and the amplification parts 14a, 14b, and 14c. The second aliquoting part 16b preferably executes a second aliquoting step of aliquoting biomolecules from the pretreatment step to the measurement step.

The amplification parts 14a, 14b, and 14c are parts that measure the biomolecules acquired by the nucleic acid extraction parts 13a and 13b. The amplification parts 14a, 14b, and 14c preferably execute the measurement step of measuring the biomolecules acquired in the pretreatment step.

Here, an example in which the nucleic acid extraction parts are configured as two nucleic acid extraction parts 13a and 13b, and the amplification parts are configured as three amplification parts 14a, 14b, and 14c is shown, but the numbers are examples and may be one or four or more. Similarly, an example in which the number of first aliquoting parts 16a and the number of second aliquoting parts 16b are each one is shown, but the numbers may be two or more.

Furthermore, the control computer 2 is connected to the genetic testing device 1 via a communication line 3.

The control computer 2 is constituted by a display unit 2a, an analysis data processing unit 2b, a control unit 2c, a calculation unit 2d, an information storage unit 2e, and the like.

The display unit 2a is a touch panel type display that also serves as an input unit, and displays various information on the genetic testing device 1, information on a sample to be analyzed, and information required to execute the analysis. A separate input device such as a mouse or a keyboard may be provided as the input unit.

The analysis data processing unit 2b is a unit that performs an analysis calculation process on the basis of measurement results obtained by the amplification parts 14a, 14b, and 14c, and the analysis data processing unit 2b preferably executes an analysis data processing step of performing an analysis calculation process on the basis of the measurement results obtained in the measurement step.

The control unit 2c is a unit that individually or collectively controls operations of the parts in the genetic testing device 1. In this example, the control unit 2c controls the operation of the first aliquoting part 16a so that an aliquoting process for a sample is performed in the order of a target time obtained in the calculation unit 2d to be described below, specifically, so that an aliquoting process for a sample is performed in the order of a target time.

The control unit 2c can also issue a warning when the target time is exceeded.

The control unit 2c executes a warning step of issuing a warning when the target time is exceeded and executes a first aliquoting step so that an aliquoting process for a sample is performed in the order of the target time.

The calculation unit 2d is a unit that calculates a target time by which the aliquoting of a sample is to be completed by adding a waiting time according to the priority of the sample to the time when the sample is fed, and the calculation unit 2d preferably executes a calculation step of calculating a target time by which the aliquoting of the sample is to be completed by adding the waiting time according to the priority of the sample to the time when the sample is fed.

The information storage unit 2e is a recording medium that stores, for example, control parameters corresponding to each unit and sample information on various samples, and also confirms and stores priority, and the information storage unit 2e preferably executes an information storage step of confirming and storing priority.

The analysis data processing unit 2b, the control unit 2c, and the calculation unit 2d of the control computer 2 may be configured as hardware using a dedicated circuit board, or may be constituted by software executed by a computer. When the analysis data processing unit 2b, the control unit 2c, and the calculation unit 2d are constituted by hardware, they can be realized by integrating a plurality of calculators that execute processing on a wiring board, or in a semiconductor chip or a package. When the analysis data processing unit 2b, the control unit 2c, and the calculation unit 2d are constituted by software, they can be realized by installing a high-speed general-purpose CPU in the computer and executing a program that executes a desired calculation process. It is also possible to upgrade existing devices using a recording medium on which this program is recorded. In addition, these devices, circuits, and computers are connected by a wired or wireless network, and data is transmitted and received as appropriate.

The above is the configuration of the genetic testing device 1 of this example.

Hereinafter, a flow of analysis in the genetic testing device 1 will be briefly described.

When a sample rack 31 with sample containers 33 installed is placed in the sample rack feeding part 11 and analysis is started, the sample rack is drawn into the sample rack buffer part 12.

The nucleic acid extraction parts 13a and 13b perform an operation of extracting nucleic acid in accordance with a requested item.

The extracted extract liquid is transferred to the amplification parts 14a, 14b and 14c and reacted with a reagent installed in a reagent installation part 15. Here, a target DNA is exponentially amplified and detected by repeating temperature control according to a thermal profile.

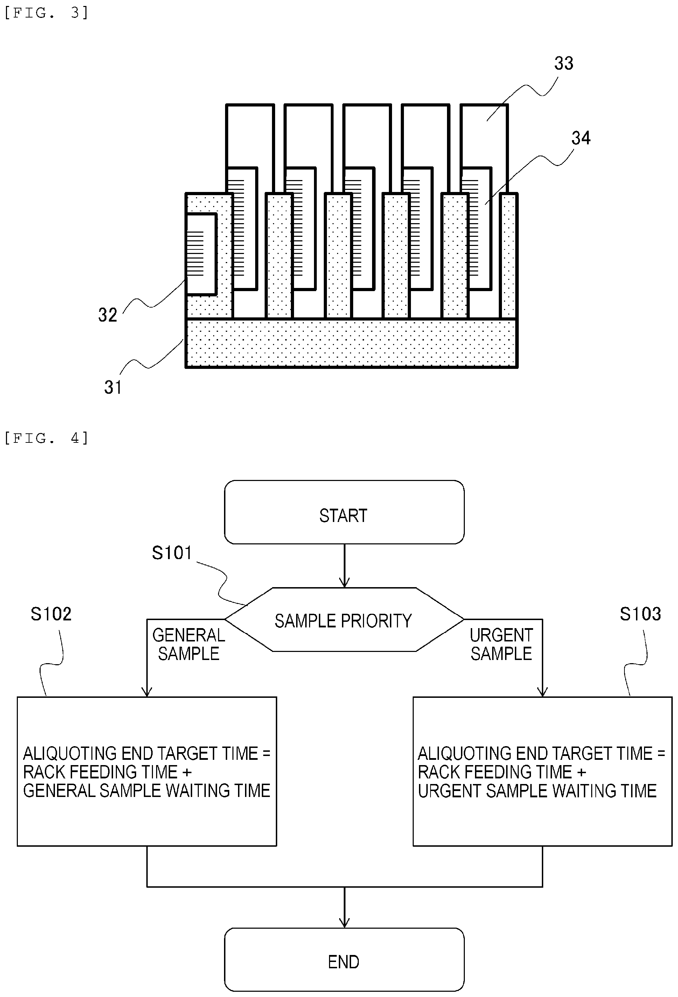

The sample containers 33 are installed in the sample rack 31 as shown in Fig. 3. In general, the sample rack 31 has a different color in accordance with the priority of the samples installed. For example, a sample rack in which urgent samples are installed is red, and a sample rack in which general samples are installed is gray. This allows a laboratory technician to easily identify a sample rack to be installed in accordance with the priority of samples when the samples are installed.

As a device, it is necessary to incorporate expensive equipment such as for image processing when directly identifying a color, and thus the priority of each sample installed on a sample rack is identified by writing information for identifying the priority of the sample rack on a rack barcode 32 and reading the information using the barcode reader 11a.

A sample barcode 34 for identifying a sample itself is attached to the sample container 33. Each sample is identified by reading the information using the barcode reader 11a.

Although not described in the text, barcode information of a sample and request information for the sample are paired and registered in a higher-level host computer such as a hospital information system (HIS). After reading the sample barcode information, the device inquires of the higher-level host computer and determines request information for the sample to be measured.

In this example, the genetic testing device using the sample rack has been described, but it is not necessary to feed a sample using the sample rack. A genetic testing device using a single sample holder or a genetic testing device configured such that a sample is directly placed by a laboratory technician may be used.

Further, in Fig. 3, five sample containers are shown to be installed in the sample rack, but the number of sample containers may be one or may be a different number such as ten, and is not particularly limited.

The priority of samples that are carried in is defined by information linked to the host computer (not shown for convenience) or sample information. In general, there are general samples that are treated as normal patient samples and urgent samples that have to be measured promptly.

In this example, two priorities are defined, but three, four, or more priorities may be provided. In this case, a configuration is adopted in which a waiting time is set for each different priority, and a target time by which aliquoting is to be completed is calculated for each priority.

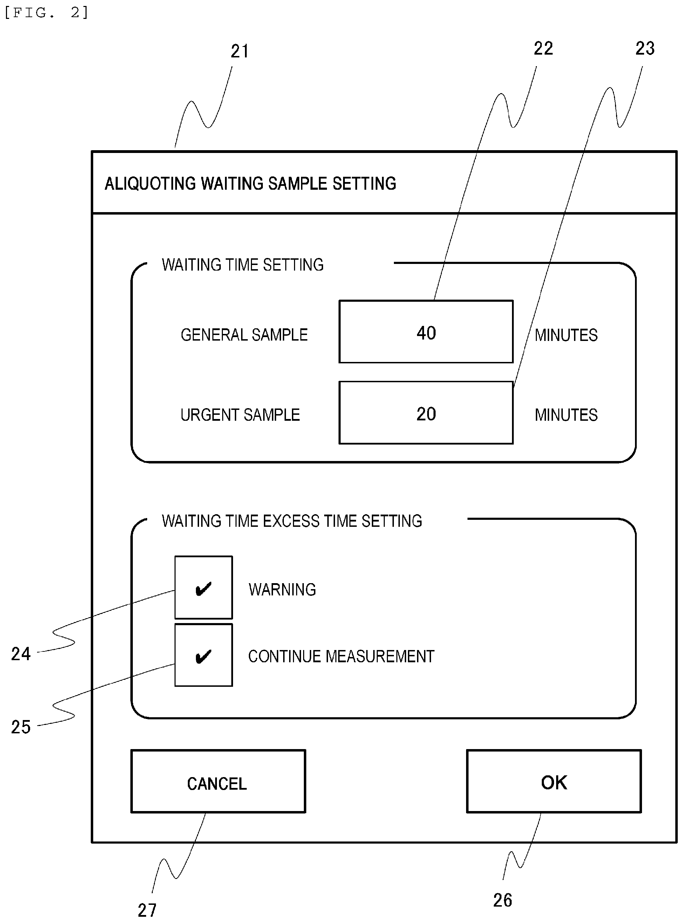

In Fig. 2, an aliquoting waiting sample setting screen 21 is displayed on the display unit 2a, in which the time that the sample can wait in the device is registered for each of these sample priorities.

On the aliquoting waiting sample setting screen 21 displayed on the display unit 2a, an input can be performed in a general sample waiting time setting field 22 for setting a waiting time for general samples in the sample rack buffer part 12, and an urgent sample waiting time setting field 23 for setting a waiting time for urgent samples in the sample rack buffer part 12. The input settings in the general sample waiting time setting field 22 and the urgent sample waiting time setting field 23 are time setting steps for setting a waiting time.

The setting of this sample setting screen 21 is set in advance by a hospital administrator or a serviceman when the device is installed.

When an OK button 26 is pressed, an input value is recorded as a waiting time, and when a cancel button 27 is pressed, the aliquoting waiting sample setting screen 21 is closed.

Next, a procedure for scheduling sample aliquoting and analysis, which is the gist of the present invention, will be described with reference to Figs. 4 to 7.

As described above, generally, a sample container containing samples is installed on the sample rack 31, the rack barcode 32 and the sample barcode 34 are read by the barcode reader 11a, and then the samples are fed into the sample rack feeding part 11. At this time, a target aliquoting end time is determined in accordance with a flow in Fig. 4.

As shown in Fig. 4, the calculation unit 2d starts a target aliquoting end time calculation process when a sample is fed.

First, the calculation unit 2d determines a sample priority (S101). When the sample priority is a general sample, the process proceeds to S102, where a waiting time of the sample, that is, the target time when aliquoting ends, is determined by a formula "target aliquoting end time" = "time when rack is fed" + "general sample waiting time (a time that is input to the general sample waiting time setting field 22)" (S102), and the process ends.

On the other hand, when the sample priority is an urgent sample, the process proceeds to S103, where a waiting time of the sample, that is, the target time when aliquoting ends, is determined by a formula "target aliquoting end time" = "time when rack is fed" + "urgent sample waiting time (a time that is input to the urgent sample waiting time setting field 23)" (S103), and the process ends.

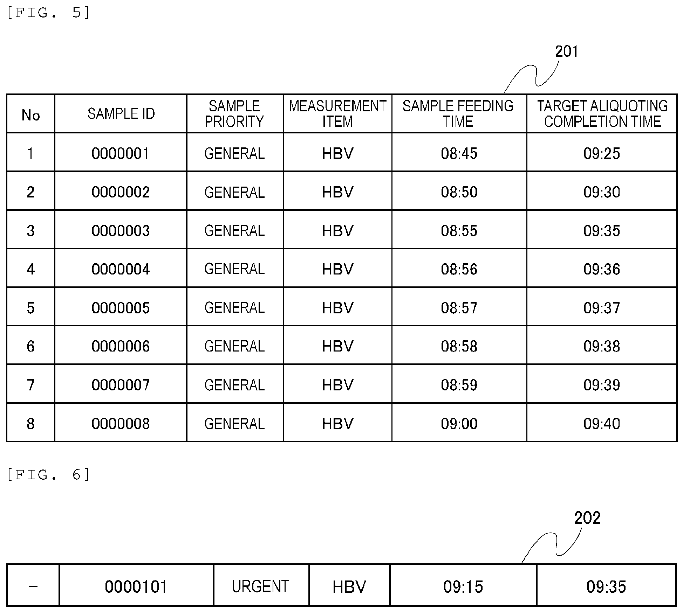

These results are reflected in an aliquoting order management table 201 in Fig. 5.

Here, it is assumed that a waiting time for general samples is set to 40 minutes, and a waiting time for urgent samples is set to 20 minutes. Since a target aliquoting completion time is determined in accordance with samples being sequentially fed from 08:45 to 09:00, the samples are arranged in order according to this time.

Thereafter, as shown in Fig. 6, when an urgent sample with a sample ID of 0000101 is carried in at 09:15, a target aliquoting completion time is calculated to be 09:35 in S103, and these pieces of information are generated as an aliquoting order management table 202.

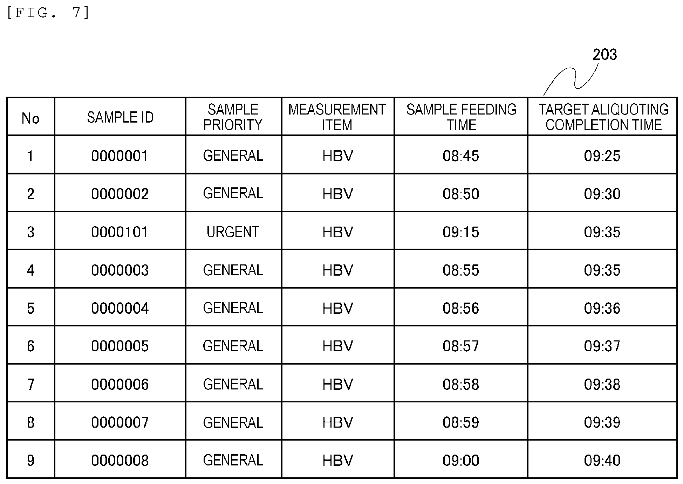

These pieces of information are added to the aliquoting order management table 201, and as shown in Fig. 7, an aliquoting order management table 203 is created after the urgent sample is carried in. Here, the target aliquoting completion time of the added urgent sample is 09:35, which is the same time as a sample ID of 0000003. When the times are the same, the sample is registered first in accordance with the sample priority. There is no problem with the logic that a sample that is fed first is given priority.

The sample fed into the device is aliquoted in accordance with the aliquoting order management table 203, and is measured after passing through the nucleic acid extraction parts 13a and 13b and the amplification parts 14a, 14b, and 14c.

In easy consideration, the aliquoting order management table 203 is created in accordance with the sample priority. In this case, an urgent sample with a sample ID of 0000101 is disposed at the top. When urgent samples arrive in succession after this, the urgent samples will be assigned before a general sample with a sample ID of 0000001, and general samples will never be measured. That is, there is a concern that the deterioration of the samples will progress.

However, in this example, a limit is also set for a waiting time for general samples, and thus it is possible to measure a sample by minimizing a delay of the general samples due to interruption while maintaining the speed of measurement by interruption of urgent samples. That is, it is possible to provide a genetic testing device that can prevent deterioration of samples that would affect the analytical performance due to waiting for a long time inside the device.

Here, in this example, the nucleic acid extraction parts 13a and 13b are considered to be two systems, and the amplification parts 14a, 14b, and 14c are considered to be three systems.

When any equipment breaks down, for example, when one of the nucleic acid extraction parts 13a and 13b breaks down and operation has to be performed with only one nucleic acid extraction part, a nucleic acid extraction process for all samples has to be processed, and thus the overall processing speed has to be reduced. In such a case, the aliquoting of samples is not necessarily completed by the target aliquoting completion time, and there is a high possibility that the time will be exceeded.

In preparation for such a situation, a warning can be issued to an operator in accordance with the setting in a waiting time excess warning setting field 24. This warning allows the operator to recognize that the set time has been exceeded.

In addition, it is possible to execute either a process of waiting for aliquoting even when the time is exceeded or a process of cancelling measurement and collecting samples in accordance with a waiting time excess measurement continuation setting field 25 for setting whether to measure the corresponding sample when the target time is exceeded.

The input setting in the waiting time excess measurement continuation setting field 25 is a setting step of setting whether to measure the corresponding sample when the target time is exceeded.

Next, effects of this example will be described.

The genetic testing device 1 of this example described above includes the sample rack feeding part 11 for feeding a sample, the nucleic acid extraction parts 13a and 13b that acquire biomolecules including a measurement target from the sample fed into the sample rack feeding part 11, the amplification parts 14a, 14b and 14c that measure the biomolecules acquired by the nucleic acid extraction parts 13a and 13b, the analysis data processing unit 2b that performs an analysis calculation process on the basis of measurement results of the amplification parts 14a, 14b and 14c, and the control unit 2c that controls the operations of the parts in the device individually or as a whole. The device further includes the calculation unit 2d that calculates a target time by which aliquoting of the sample is to be completed by adding a waiting time according to the priority of the sample to the time when the sample is fed, and the control unit 2c performs an aliquoting process for the sample in the order of the target times.

In such the genetic testing device 1, samples are processed in order of shortest expiration date, and thus the measurement of samples with higher priority can be prioritized. On the other hand, since processing is not performed only by priority, it is possible to avoid having to continuously wait for measurements of low-priority samples even when high-priority samples are fed successively.

In addition, since the general sample waiting time setting field 22 and the urgent sample waiting time setting field 23 for setting a waiting time are further provided, it is possible to change a waiting time as needed in accordance with the operating status of the device and the actual situation of the operator and to realize more flexible analysis.

In addition, the barcode reader 11a that reads the priority of a sample when the sample is fed is further provided, and thus a user does not need to manually input the priority each time, making it possible to reduce the burden.

In addition, the control unit 2c can determine whether it is necessary to take measures such as recollecting or refeeding of samples by issuing a warning when a target time is exceeded, and can obtain accurate analysis results even when a problem occurs.

Furthermore, the waiting time excess measurement continuation setting field 25 for setting whether to perform measurement of the corresponding sample when a target time is exceeded is further provided, and thus it is possible to set whether to analyze, as it is, a sample that is likely to deteriorate due to a long waiting time in accordance with the operating status of the device and the actual situation of the operator and to realize more flexible analysis.

Furthermore, the device further includes the first aliquoting part 16a that aliquots samples from the sample rack feeding part 11 to the nucleic acid extraction parts 13a and 13b, and the second aliquoting part 16b that aliquots biomolecules from the nucleic acid extraction parts 13a and 13b to the amplification parts 14a, 14b, and 14c, and the control unit 2c controls the operation of the first aliquoting part to perform a sample aliquoting process in the order of a target time, and thus the operation of the first aliquoting part 16a that directly aliquots a sample having the greatest effect on sample deterioration can be set as an operation of prioritizing the measurement of a sample with a high priority and avoiding having to continuously wait for measurements of low-priority samples.

### <Others>

The present invention is not limited to the above-described example, and various modifications and applications can be made. The above-described example is described in detail to describe the present invention in an easy-to-understand manner, and is not necessarily limited to having all of the configurations described.

### Reference Signs List

1: genetic testing device
2: control computer
2a: display unit
2b: analysis data processing unit
2c: control unit
2d: calculation unit
2e: information storage unit
3: communication line
11: sample rack feeding part (feeding part)
11a: barcode reader
12: sample rack buffer part
13a, 13b: nucleic acid extraction part (pretreatment part)
14a, 14b, 14c: amplification part (measurement unit)
15: reagent installation part
16a: first aliquoting part
16b: second aliquoting part
21: aliquoting waiting sample setting screen
22: general sample waiting time setting field
23: urgent sample waiting time setting field
24: waiting time excess warning setting field
25: waiting time excess measurement continuation setting field (setting unit)
26: OK button
27: cancel button
31: sample rack
32: rack barcode
33: sample container
34: sample barcode
201, 202, 203: aliquoting order management table

## Claims

1. A genetic testing method comprising:
a feeding step of feeding a sample;
a pretreatment step of acquiring biomolecules including a measurement target from the sample fed in the feeding step;
a measurement step of measuring the biomolecules acquired in the pretreatment step;
an analysis data processing step of performing an analysis calculation process based on a measurement result in the measurement step; and
a calculation step of calculating a target time by which aliquoting of the sample is to be completed by adding a waiting time according to priority of the sample to a time when the sample is fed,
wherein an aliquoting process for the sample is performed in the order of the target time.

2. The genetic testing method according to claim 1, further comprising a time setting step of setting the waiting time.

3. The genetic testing method according to claim 1, further comprising a reading step of reading the priority of the sample when the sample is fed.

4. The genetic testing method according to claim 1, further comprising an information storage step of confirming and storing the priority.

5. The genetic testing method according to claim 1, further comprising a warning step of issuing a warning when the target time is exceeded.

6. The genetic testing method according to claim 1, further comprising a setting step of setting whether to measure the corresponding sample when the target time is exceeded.

7. The genetic testing method according to claim 1, further comprising:
a first aliquoting step of aliquoting the sample in the feeding step to the pretreatment step; and
a second aliquoting step of aliquoting the biomolecules in the pretreatment step to the measurement step,
wherein the first aliquoting step is executed to perform the aliquoting process for the sample in the order of the target time.

8. A genetic testing device comprising:
a feeding part into which a sample is fed;
a pretreatment part that acquires biomolecules including a measurement target from the sample fed into the feeding part;
a measurement unit that measures the biomolecules acquired by the pretreatment part;
an analysis data processing unit that performs an analysis calculation process based on a measurement result in the measurement unit;
a control unit that controls operations of the parts in the device individually or collectively; and
a calculation unit that calculates a target time by which aliquoting of the sample is to be completed by adding a waiting time according to priority of the sample to a time when the sample is fed,
wherein the control unit performs an aliquoting process for the sample in the order of the target time.

9. The genetic testing device according to claim 8, further comprising a time setting unit that sets the waiting time.

10. The genetic testing device according to claim 8, further comprising a reading unit that reads the priority of the sample when the sample is fed.

11. The genetic testing device according to claim 8, further comprising an information storage unit that confirms and stores the priority.

12. The genetic testing device according to claim 8, wherein the control unit issues a warning when the target time is exceeded.

13. The genetic testing device according to claim 8, further comprising a setting unit that sets whether to measure the corresponding sample when the target time is exceeded.

14. The genetic testing device according to claim 8, further comprising:
a first aliquoting part that aliquots the sample from the feeding part to the pretreatment part; and
a second aliquoting part that aliquots the biomolecules from the pretreatment part to the measurement unit,
wherein the control unit controls an operation of the first aliquoting part to perform the aliquoting process for the sample in the order of the target time.
